# EUROPEAN PATENT SPECIFICATION

(11) **EP 2 228 383 B1**
(45) Date of publication and mention of the grant of the patent: **23.05.2012**
(21) Application number: 10159280.6
(22) Date of filing: 26.03.2004
(51) Int. Cl.: C07K 14/245

(54) **Library of toxin mutants, and methods of using same**
Bibliothek von Toxin Mutanten und deren Verwendung
Bibliothèque de mutants de toxines et procédés pour leur utilisation

(43) Date of publication of application: 15.09.2010
(62) Divisional of application: 04785852.7
(73) Proprietor: Molecular Templates, Inc., Georgetown, TX 78626 (US)
(72) Inventor: Gariepy, Jean, Toronto, Ontario M5P 2 L5 (CA); Wei, Xin, Markham, Ontario L6E 1R9 (CA)
(74) Representative: Vossius & Partner

(56) References cited:
- WO-A-99/40185
- US-A- 5 552 144
- BRAY MARK R ET AL: "Probing the surface of eukaryotic cells using combinatorial toxin libraries" CURRENT BIOLOGY, vol. 11, no. 9, 1 May 2001 (2001-05-01), pages 697-701, XP002303223 ISSN: 0960-9822
- BRAY ET AL: "Shiga-like toxin as a template for the development of anti-breast cancer agents" PROCEEDINGS OF THE 89TH. ANNUAL MEETING OF THE AMERICAN ASSOCIATION FOR CANCER RESEARCH. NEW ORLEANS, LA, MARCH 28 - APRIL 1, 1998, PROCEEDINGS OF THE ANNUAL MEETING OF THE AMERICAN ASSOCIATION FOR CANCER RESEARCH, PHILADELPHIA, PA : AACR, US, vol. VOL. 39, 1 March 1998 (1998-03-01), page 62, XP002096335
- BRAY ET AL: "Expression of the Shiga-like toxin I receptor CD77 on human breast carcinomas, follicular lymphomas and multiple myelomas and absence of expression on CD34+ human hematopoietic cells: Implications for tumor cell purging" PROCEEDINGS OF THE 89TH. ANNUAL MEETING OF THE AMERICAN ASSOCIATION FOR CANCER RESEARCH. NEW ORLEANS, LA, MARCH 28 - APRIL 1, 1998, PROCEEDINGS OF THE ANNUAL MEETING OF THE AMERICAN ASSOCIATION FOR CANCER RESEARCH, PHILADELPHIA, PA : AACR, US, vol. VOL. 39, 1 March 1998 (1998-03-01), page 63, XP002096336

## Description

### Background of the Invention

This application relates to libraries of toxin mutants, and to methods of using same in the development of therapeutics targeted against specific cell types.

Plant and bacterial toxins have a structural organization with two or more polypeptide domains or subunits responsible for distinct functions, referred to as A and B. The toxins may be referred to as ABₓ toxins where x represents the number of identical or homologous B subunits in the toxin. This family of framework-related toxins includes examples such as Shiga and Shiga-like toxins, the E. coli heat-labile enterotoxins, cholera toxin, diphtheria toxin, pertussis toxin, Pseudomonas aeruginosa exotoxin A (Olsnes, S. and Sandvik, K. (1988) in immunotoxius pp. 39-73, Kluwer Academic, Boston; Sandvik, K., Dubinina, E., Garred, O., et al. (1992) Biochem. Soc. Trans. 20:724) as well as plant toxins such as ricin and abrin. In some cases the toxin are heteromeric, in that the B chains are actually separate entities that connect to the toxic A chain via a non-covalent bonding. In other cases, the toxin is monomeric, since the B chain is part of the same protein when the toxin is produced in nature.

Based on their ability to block protein synthesis, proteins such as Shiga and Shiga-like toxins as well as ricin, abrin, gelonin, crotin, pokeweed antiviral protein, saporin, momordin, modeccin, sarcin, diphtheria toxin and exotoxin A have been referred to as ribosome-inactivating proteins (RIP). The potency of RIPs is exceedingly high; one molecule of diphtheria toxin A chain (Yamaizumi, et al. (1978) Cell 15:245-250) or ricin A chain (Eiklid, et al. (1980) Exp. Cell Res. 126:321-326) having been shown to be sufficient to kill a eukaryotic cell.

International Patent Publication No. WO99/40185 describe libraries of mutant toxins in which mutations are introduced into the binding domain to alter the type of cells to which the toxic species are delivered. The new proteins are derived by mutating a binding subunit of the wild type heteromeric protein cytotoxic protein to create a library of microorganism clones producing mutant proteins, which are then screened for the ability to specifically bind to and kill a target cell type.

US Patent No. 5,552,144 discloses a Shigella-like toxin II variant to which a mutation is introduced into the A chain at position 167 to change the amino acid at this position to one with a different charge. This resulted in a toxin with less of the enzymatic activity associated with toxicity.

US Patent No. 6,593,132 describes recombinant toxic proteins which are specifically toxic to diseased cells but do not depend for their specificity of action on a specific cell binding component. The recombinant proteins of the 132 patent have an A chain of a ricin-like toxin linked to a B chain by a synthetic linker sequence which may be cleaved specifically by a protease localized in cells or tissues affected by a specific disease to liberate the toxic A chain thereby selectively inhibiting or destroying the diseased cells or tissues.

US Patent No. 6,649,742 disclosed Type I ribosome-inactivating proteins (RIPs) and analogs of the RIPs having a cysteine available for disulfide bonding to targeting molecules. The RIPs and RIP analogs are used as components of cytotoxic therapeutic agents to selectively eliminate any cell type to which the RIP component is targeted by the specific binding capacity of the second component of the agent.

### Summary of the Invention

The present provides combinatorial protein libraries comprising a plurality of protein species, in which each protein species comprising an A chain of a heteromeric toxic protein into which an insert has been introduced. In accordance with the invention, the insert is a polypeptide of varying amino acid sequence having a length of 2 or more amino acid residues, for example from 3 to 200 amino acid residues; and the insert is introduced into the protease-sensitive loop of the A chain sequence. The result of the introduction of the insert creates an artificial binding domain within the A chain, such that the A chain develops toxic specificity which is independent of and different from the normal specificity associated with the B chains binding domains. Screening of the library allows the selection and identification of mutant toxins that are specific for different cell types, including cancer cell types. In one embodiment of the invention, the combinatorial library comprises protein species that are formed by introducing the insert into a Shiga-like toxin I A chain, for example in the region between amino acids 242 and 261, as defined with reference to Seq. ID No. 1.

The invention also provides a combinatorial expression library comprising a plurality of species of expression systems, each species expressing a protein species comprising an A chain of a heteromeric toxic protein into which the insert as described above has been introduced. Expression of protein from the combinatorial express on library results in the formation of a combinatorial protein library.

In a further aspect, the invention provides a composition for treating melanoma, and methods for using such a composition. The composition comprises a protein species comprising an A chain of a heteromeric toxic protein into which a polypeptide having a length of 2 or more amino acid residues, for example from 3 to 200 amino acid residues, is introduced into the protease-sensitive loop of the A chain sequence. The insert is selected such that protein species has toxic activity towards melanoma cells. The protein species is used in the treatment of melanoma by administering it to a patient diagnosed with melanoma, in an amount sufficient to result in the reduction of the number of living melanoma cells.

In a further aspect, the invention provides a composition for treating other types of cancer. The composition comprises a protein species comprising an A chain of a heteromeric toxic protein into which a polypeptide having a length of 2 or more amino acid residues, for example from 3 to 200 amino acid residues, is introduced into the protease-sensitive loop of the A chain sequence. The insert is selected such that protein species has toxic activity towards the cancer cells. In a specific embodiment, the insert is selected to bind to MUC-1 receptors. The protein species is used in the treatment of melanoma by administering it to a patient diagnosed with melanoma, in an amount sufficient to result in the reduction of the number of living melanoma cells.

In a further aspect, the invention provides a method for identifying ligands that bind to specific targets/receptors, such as tumor markers known to exist on cancer cells. In this method, the toxin of the combinatorial library serves as a reporter, and a combinatorial protein library in accordance with the invention is screened against cells known to possess the target/receptor. Proteins which are shown to be toxic to the cells are evaluated to determine the sequence of the inserted region Peptides of this sequence can then be used, in combination with a toxin or other molecules, to direct compounds to cells possessing the target/receptor.

In yet a further aspect of the invention, a method is provided for identifying toxic substances specific for a known cell marker. In this embodiment of the invention, the toxin need not serve as reporter. Thus, cells having the marker, or an isolated target/receptor, where available, are exposed to the combinatorial protein library. In preferred embodiments, the cells or the isolated target/receptor are immobilized on a solid support, such as in plastic wells. Captured proteins from the library are then rescreened against cells to confirm their toxicity and specificity for cells expressing the target/receptor, and their suitability for use as a therapeutic.

### Brief Description of the Drawings

Fig. 1 is a schematic diagram depicting the A1 and A2 domains of SLT-1. The A chain is composed of 293 amino acids. The chain is cleaved by furin to produce an A1 catalytic fragment and an A2 C-terminal tail non-covalently associated with the B pentamer. A protease-sensitive loop (stripped area) is defined by the only two cysteine residues in the A chain (Cys 242 and 261). Tyr77, Glu167, Arg170 and Trp203 represent residues crucial for the catalytic activity of the A1 domain (arrows).
Fig. 2A is a schematic representation of the SLT-1 A chain (1-293) with the breast cancer-associated MUC1 epitope PDTRPAP (control sequence recognized by the mAb One M27) inserted between residues 245 and 246 and a 6-Histidine tag at its N-terminus.
Fig. 2B is a depiction of our SLT-1 A chain-tripeptide library construction where the three key positions of the MUC1 epitope recognized by the mAb One M27 were randomized (XXX region). The tripeptide library was inserted in a naturally occurring loop region of the A chain created by the presence of a disulfide bridge between Cys 242 and Cys 261.
Fig. 3 shows a representative ELISA data set from screening 96 distinct single A chain variants from our SLT-1 A chain-tripeptide library with mAb One M27. Toxin variant # 41 (Tables 1 and 2) gave a strong ELISA signal and had the expected epitope.
Fig. 4 shows a schematic diagram of a 7-amino acid random segment inserted between residues 245 and 246 of the A chain.
Fig. 5 shows the results of tests on seven toxin variants that: were identified as repeatable killers of the human melanoma cell line 518A2. The abscissa represents the log concentration of toxin used to treat the cells and the ordinate depicts the observed percentage of cells that are viable after 48 hours. The closed triangles depicts the effect of the wild type toxin on 518A2 cells while the two most efficacious A chain variants were termed SAM#3 (open squares) and SAM#5 (X symbols).

### Detailed Description of the Invention

The present invention relates to a combinatorial protein library comprising a plurality of protein species, each protein species comprising an A chain of a heteromerie toxic protein into which an insert has been introduced. The insert is a polypeptide of varying amino acid sequence having a length of 2 or more amino acid residues, for example from 3 to 200 amino acid residues; and is introduced into the protease-sensitive loop of the A chain sequence. The library provides a collection of protein species that can be screened for individual proteins that are toxic against specific cell types, such as specific cancer cell types. individual protein species thus selected are suitably used in the treatment of the cancer.

As used in the specification and claims of this application, the term "combinatorial library" refers to a mixture of species each of which has a common portion and a variable portion. In the case of a "combinatorial protein library" each of the species is a protein or peptide, and the common portions and the variable portions are each amino acid sequences. In the case of a combinatorial expression library, the species are microorganism, expression vectors or polynucleotides which, when expressed, produce proteins or peptides having common portions and variable portions. In this case, the common portions and the variable portions are each nucleotide sequences. Since the purpose of the combinatorial library is to provide multiple variants for screening purposes, the combinatorial library preferably contains at least 100 distinct species of protein or expression unit, more preferably at least 1000 distinct species.

As used in the specification and claims of this application, the term "heteromeric toxic protein" refers to the class of protein toxins with the common organization theme of being heteromeric in nature with two or more polypeptide domains or subunits responsible for distinct functions (Merritt, E.A., and Hol, W.G.J. (1995) Curr. Opin. Struct. Biol. 5:165 1). In such proteins, the two or more subunits or domains could be referred to as A and B, and the toxins as ABₓ toxins where x represents the number of identical or homologous B subunits in the toxin. This family of framework-related toxins includes examples such as Shiga and Shiga-like toxins, the E. coli heat-labile enterotoxins, cholera toxin, diphtheria toxin, pertussis toxin, Pseudomonas aeruginosa exotoxin A as well as plant toxins such as ricin and abrin Based on their ability to block protein synthesis, proteins such as Shiga and Shiga-like toxins as well as ricin, abrin, gelonin, crotin, pokeweed antiviral protein, saporin, momordin, modecein, sarcin, diphtheria toxin and exotoxin A have been referred to as ribosome-inactivating proteins (RIP). In these naturally-occurring heteromeric toxic proteins, the A chain is the toxic portion, while the B chains form a binding moiety which binds to a receptor on a cell susceptible to the toxin, thereby delivering the A chain to the cell.

One specific example of the A chain of a heteromeric toxic protein is the A chain of SLT-1 which has the sequence given is Seq. ID No. 1. The A chain of SLT-1 comprises of 293 amino acids with the enzymatic (toxic) domain spanning residues 1 to 239. A protease sensitive loop encompassing residues 242 to 251 is normally exposed, and is a suitable site for inserting a peptide sequence.

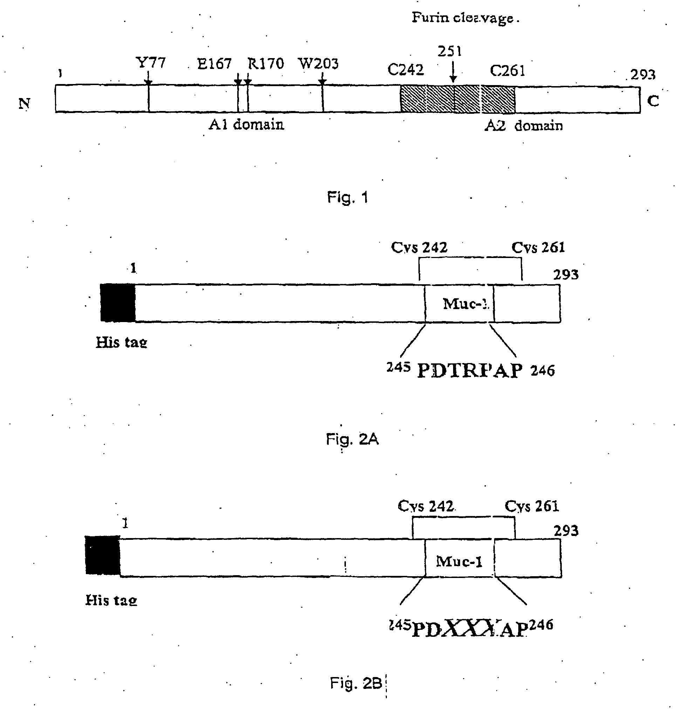

SLT-1 is a type II ribosome inactivating protein produced by pathogenic strain of Escherichia coli (0157:H7) (24). SLT-1 is an AB5 complex of about 70 kD (OBrien, A. D., and Holmes, R. K. (1987) Shiga and Shiga-like toxins. Microbiol Rev 51, 206-220.). The single 32 kD catalytic A subunit is non-covalently associated with a pentamer of five identical 7.7 kD B subunits. The B subunit pentamer recognizes the glycolipid globotriaosylceramide (also known as CD77 or Gb3) on the surface of target cells (Lingwood, C. A. (1993) Verotoxins and their glycolipid receptors. Adv Lipid Res 25, 189-211; Jacewicz, et al. (1986) Pathogenesis of shigella diarrhea. XI. Isolation of a shigella toxin-binding glycolipid from rabbit jejunum and HeLa cells and its identification as globotriaosylceramide. J Exp Med 163, 1391-1404). A protease-sensitive loop located between Cys242 and 261 at the C terminus of the A chain is cleaved by furin during cellular routing (Fig. 1). The A chain remains associated with its B subunit pentamer due to an intrachain disulfide bond between Cys242 and Cys261 as it travels to the ER lumen (Sandvig, et al. (1989) Endocytosis from coated pits of Shiga toxin: a glycolipid-binding protein from Shigella dysenteriae 1, J Cell Biol 108, 1331-1343;32. Garred, et al. (1995) Role of processing and intracellular transport for optimal toxicity of Shiga toxin and toxin mutants. Exp Cell Res 218, 39-49.). The disulfide bond is finally reduced in the ER lumen and the A1 chain (first 251 aa) is released and subsequently retrotranslocated to the cytosol where it inactivates ribosomes (O'Brien, et al. (1992) Shiga toxin: biochemistry, genetics, mode of action, and role in pathogenesis. Curr Top Microbiol Immunol 180, 65-94.). More specifically, the A chain of SLT-1 is a N-glycosidase that catalytically cleaves a specific adenine nucleotide (4324) from the 28 S rRNA (Brigotti, et al. (1997) The RNA-N-glycosidase activity of Shiga-like toxin I: kinetic parameters of the native and activated toxin. Toxicon 33,1431-1437.). This event leads to the inhibition of protein synthesis by preventing the binding of aminoacyl tRNAs to the ribosome and halting protein elongation. Mutagenesis studies as well as structural analysis performed on the A chains of ST and ricin have delineated key conserved residues involved in catalytic activity (Deresiewicz, et al.(1992) Mutations affecting the activity of the Shiga-like toxin I A-chain. Biochemistry 31, 3272-3280; Ready, et al. (1991) Site-directed mutagenesis of ricin A-chain and implications for the mechanism of action. Proteins 10, 270-278). Residues crucial for catalytic activity of SLT-1 are tyrosine 77, glutamic acid 167, arginine 170 and tryptophan 203 (Hovde, et al. (1988) Evidence that glutamic acid 167 is an active-site residue of Shiga-like toxin I. Proc Natl Acad Sci U S A. 85, 2568-2572; Yamasaki, et al. (1991) Importance of arginine at position 170 of the A subunit of Vero toxin 1 produced by enterohemorrhagic Escherichia coli for toxin activity. Microb I'athog 11, 1-9). In addition, binding of the toxin to the cell surface is critical to introduction into the cell and thus for toxic activity. Because of this the A chain alone is not significantly toxic.

In addition to the A chain of SLT-1, other toxins may also be used to form libraries and compositions in accordance with the invention, and may be used in the methods of the invention. Specifically, Shiga and other Shiga-like toxins, as well as ricin, abrin, gelonin, crotin, pokeweed antiviral protein, saporin, momordin, modeccin, sarcin, diphtheria toxin and exotoxin A, and other functionally-related ribosome inactivating proteins (RIP) may be used.

For purposes of making the combinatorial library of the invention, short amino acid sequences of at least 2 amino acids, for example of 3 to 200 amino acids residues in length, are inserted into this protease sensitive loop. The number of amino acids in the insert defines the number of possible random variants that can be in the library. For example, when the number of amino acids in the insert is 3, the maximum number of variants is 20³ or 8000 variants. Larger inserts provide corresponding larger numbers of possible variants.

As an alternative to using inserts with purely random sequences, inserts can be designed based on a known template. For example, as described below in the context of Muc-1, variations in a sequence known to provide receptor binding properties for a particular cell type can be used to identify an insert that provides for optimization of the toxic properties of the protein construct. This same optimization may be performed on an individual sequence isolated by screening of a larger combinatorial library. It will be appreciated, however, that the insert in the proof of principle tests of Example 1 use an insert which is the target/receptor, while in the actual case the insert would be based on the sequence of a known ligand to be optimized for maximum effectiveness and specificity.

This approach, in which a specific, known receptor type is targeted illustrates a further aspect of the invention, namely a method for identifying peptide ligands that bind to specific targets/receptors, such as tumor markers known to exist on cancer cells. In this method, a combinatorial protein library in accordance with the invention is screened against cells known to possess the target/receptor. The toxin serves as the reporter, such that proteins which are shown to be toxic to the cells are evaluated to determine the sequence of the inserted region. Peptides of this sequence can then be used, in combination with a toxin or other molecules, to direct compounds to cells possessing the target/receptor. Further Testing peptides of the sequence of the inserted region may be appropriate to confirm that they are a ligand for the specific target/receptor, as opposed to some other receptor on the cell type. This can be done using binding assays with isolated receptor, where such are available.

The invention also provides a method for identifying toxic substances specific for a known cell marker, and particularly markers that are available in isolated form. In this embodiment of the invention, the toxin need not serve as reporter. Thus, cells having the marker, or an isolated target/receptor, where available, are exposed to the combinatorial protein library. In preferred embodiments, the cells or the isolated target/receptor are immobilized on a solid support, such as in plastic wells. Captured proteins from the library are then rescreened against cells to confirm their toxicity and specificity for cells expressing the target/receptor, and their suitability for use as a therapeutic. This method can be used to identify toxins with binding inserts specific for any rumor marker or cell receptor, including without limitation tumor markers such as mucins such as MUC-1 and its glycoforms, Her-2, Her2-Neu, tyrosine kinase markers, EGFR, GD2, and GD3.

Thus, in accordance with this specific aspect of the invention, a method for isolating a toxin specific for a known target/receptor is provided that comprises the steps of:
(a) exposing the target/receptor to a combinatorial protein library comprising a plurality of protein species, each protein species comprising an A chain of a toxic protein int which an insert has been introduced, wherein.
   the insert is a polypeptide of varying amino acid sequence having a length of least 2 amino acid residues; and
   the insert is introduced into the protease-sensitive loop of the A chain sequence; and
(b) isolating at least one protein species from the combinatorial protein library captured by binding to the target/receptor. As used in the specification and claims hereof, the term "isolating" refers to any mechanism for obtaining a composition containing the protein separated from the milieu in which it is expressed in a form suitable for further analysis. This would include release from the target/receptor following capture (for example by exposure to a competitive binding agent) or isolation from a culture of a clone expressing the protein found to be captured. The method may further comprises the step of screening the isolated protein against cells expressing the target/receptor, to confirm their toxicity for cells ex-pressing the target/receptor. Procedures suitable for this screening are described in Example 3. As noted above, in this method, the target/receptor may be a purified target/receptor and may be immobilized on a solid support. The target/receptor may also be on the surface of cells, which may be immobilized. Where the target/receptor is on the surface of cells, the toxin can serve as a reporter, and the death of the cells is indicative of receptor binding.

Our experience with constructing SLT-1 libraries has pointed out a number of practical issues that are appropriately considered in selecting a protein template for building combinatorial libraries. An important factor is to choose a single chain protein, preferably a bacterial protein of less than 300 amino acids (if the libraries are to be expressed in prokaryotes). Use of a smaller toxin both increases its potential to penetrate into solid tumors and reduces its immunogenicity. Secondly, the protein template should spontaneously fold in solution into its active form such that there is minimal need for host's chaperones. One should avoid for example scaffolds that contain multiple cysteine residues normally involved in disulfide bridges. In addition, a single chain protein as opposed to a multi-subunit complex may be more easily exported from bacteria. Thirdly, the protein template should possess an enzymatic activity, which can rapidly be measured to confirm the proper folding of peptide variants containing single and multiple site-directed mutations. Fourthy, a simple screening approach should be incorporated into the design of combinatorial libraries. Such searches should be amenable to high-throughput screening methods. The catalytic A chain of SLT-1 (residues 1 to 293) meets these criteria because it is a single chain that lacks any known receptor binding function, and that has a well defined structure and catalytic site.

A further aspect of the invention is a combinatorial expression library comprising a plurality of species of expression systems. Each species within the expression library expresses a protein species comprising an A chain of a heteromeric toxic protein into which an insert has been introduced. The insert is a polypeptide of varying amino acid sequence having a length of 2 or more amino acid residues, for example from 3 to 200 amino acid residues; and is introduced into the protease-sensitive loop of the A chain sequence. Suitable expression systems include plasmids and viral vectors.

The invention will now be further described with reference to the following, nonlimiting examples.

### Example 1

### Proof-or-concept: design and mining of a prototypic A chain-tripeptide library

We originally created a simple tripeptide library inserted in the C-terminal protease-sensitive loop of the SLT-1 A chain (Figs. 2A and B). This A chain loop region is naturally constrained due to the presence of a single disulfide bond bridging Cys242 to Cys261. The maximal diversity of this library can thus be calculated to be 20³ or 8000 permutations of a tripeptide sequence. As a proof-of-concept that A chain libraries can easily be screened for a new receptor-binding activity, we picked more than 3000 colonies from this A chain-tripeptide library and purified the mutant toxin produced by each clone. We noticed very early in this study that the level of expression of A chain mutant was dramatically increased when expressed in the presence of the wild-type SLT-1 B subunit. Thus the mutant forms of A chain were expressed and initially purified as AB5 toxin variants. Since all A subunits harbor a polyHis purification tag, it is relatively easy to remove the B subunit with denaturants (urea for example) while recovering the A chain on metal-affinity columns or beads. Western blots performed on randomly selected bacterial clones indicated that > 70% of these colonies produced significant amounts of these A chain mutants.

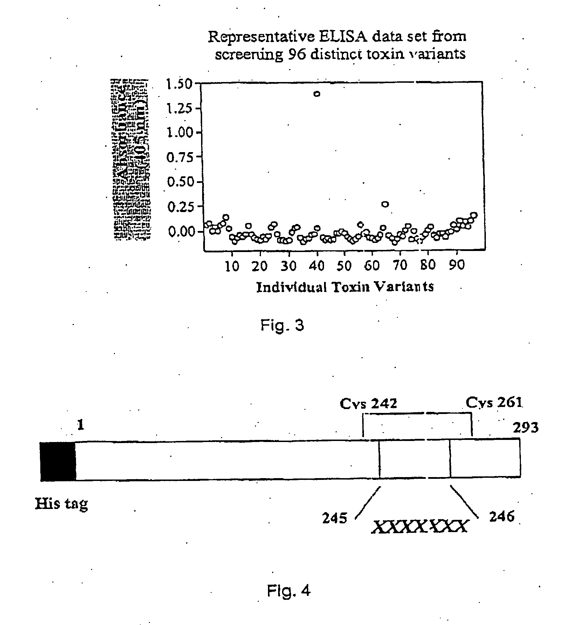

These toxin variants were then coated in individual wells of 96-well plates and screened by ELISA for their ability to bind the monoclonal antibody One M27 (Linsley, et al.(1988) Monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibody SM-3 is selectively exposed in a range of primary carcinomas. Int J Cancer 43, 1072-1076). As shown in Tables 1 and 2, most A chain mutants neither coded for the tripeptide insert that matched the targeted epitope of One M27 nor were recognized by the antibody. However on two occasions, a toxin variant harboring the exact epitope (Table 1, Fig.3) gave a strong ELISA signal comparable to the one observed with our control A chain harboring the MUC1 tripeptide epitope and had the expected epitope sequence in its randomized tripeptide region. A typical ELISA data set for 96 A chain mutants is presented in Fig. 3, highlighting the fact that the majority of A chain mutants did not recognized the mAb One M27 except for an A chain variant (mutant #41 in Tables 1 and 2). These results clearly established that A chain libraries can easily be constructed and screened to find toxin variants able to specifically target a given receptor, in this case an antigen-combining site.

### Example 2

### Making of Combinatorial SLT-1 A-heptapeptide Library

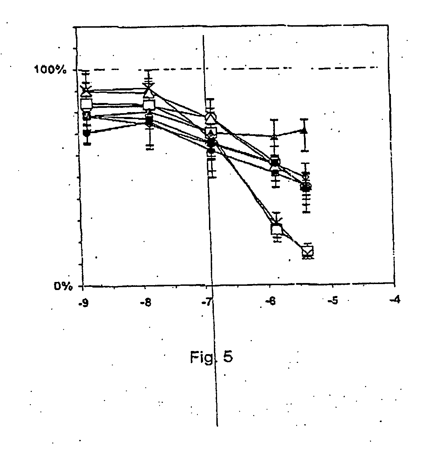

Library diversity represents a crucial parameter in screening combinatorial libraries for ligands able to bind specifically and with high affinity to a particular target. The SLT-1 A chain-tripeptide library described in Example 1 has a maximal diversity of 20³ or 8000 possible mutated A chains. This sampling repertoire is small but was useful in mapping a tripeptide epitope to establish our proof-of-concept. A seven-residue library (20⁷ or 1.3 x 10⁹ possible mutants) represents a more typical minimal diversity level commonly used in designing phage display as well as synthetic peptide libraries. Thus as a starting point, we built a SLT-1 A chain library with a 7-amino acid long random sequence inserted in its C-terminus. This library was installed in the A chain's protease sensitive loop, a loop region naturally constrained by a disulfide bond. This library provided sufficient diversity to insure that A chain toxin variants can be identified that target new or known internalized receptors on cancer cells. All elements of this library (as well as all other libraries proposed) contain a N-terminal His tag to quickly purify A chain mutants. The library (Fig. 4) was generated using a megaprimer PCR strategy (Sarkar G, and Sommers S, (1990). The 'megaprimer' method of site-directed mutagenesis. Biotechniques 8, 404-407). The megaprimer strategy is widely used to introduce mutations into target DNA sequence, by way of two rounds of PCR that use two flanking primers and an internal mutagenic primer. A description of the library design for the SLT-1 A chain-heptapeptide library will serve as the example for other future single A chain libraries. The SLT-1 A chain-heptapeptide library (Fig. 5) harbors a 7-amino acid random insertion between amino acid 245 and 246 of the A chain, the identical site used to construct our SLT-1 A chain-tripeptide library (Tables 1, 2, Fig 3). Briefly, two flanking primers A (GTT ACT GTG ACA GCT GAA GCT TTA CGT TTT CG (Seq. ID No. 2) and B (GAG AAG AAG AGA CTG CAG ATT CCA TCT GTT G (Seq. ID No. 3)) carrying Hind111 and Pst1 restriction sites respectively were annealed within the 5' and 3' ends of the SLT-1 operon. A library oligonucleotide F containing all seven random amino acid (NNS) as well as a long matching sequence to anneal to the template were sythesized. In the synthesis of the random oligonucleotide, the relative representation of each amino acid was improved by restricting the third position of each codon to G or T (Noren, K. A., and Noren, C. J. (2001) Construction of high-complexity combinatorial phage display peptide libraries. Methods 23, 169-178.). This type of restriction reduces the overall DNA sequence complexity as well as coding discrepancy between residues (Reidhaar-Olson, et al. (1991) Random mutagenesis of protein sequences using oligonucleotide cassettes. Methods Enzymo 208, 564-586). This strategy also minimizes the occurrence of stop codons (TAA and TGA while the stop codon (TAG) is suppressed by using a supE bacterial strain, which specifies for the insertion of a Gln residue when TAG codons are translated. The first PCR reaction was performed using primers A and F and the resulting product purified by denaturing polyacrylamide gel electrophoresis. This product then served as the megaprimer with primer B for a second PCR reaction to amplify the random DNA. The final library DNA (PCR product) will then be digested with Hind111 and Pst1 and cloned into the backbone of a pECHE9a expression vector (MTI, Toronto). The E. coli strain JM101 was subsequently transformed with the resulting pECHE vector and single bacterial colonies picked, lysed and their supernatants analyzed for the expression of single A chain toxins or layered on cancer cells and screened using the SRB for cell cytotoxicity assay.

### Example 3

### Mining the combinatorial SLT-1 A-beptapeptide library against cancer cell lines using a cytotoxicity assay

We screened our SLT-1 A-heptapeptide library using the cytotoxic function of the A chain as a reporter signal. Cytotoxicity is a more informative properly to measure than binding to a receptor since it implies that the toxin is internalized, processed and delivered near ribosomes, clearly a multi-step event. The cytotoxicity assay was essentially performed as previously described (Bray, et al, (2001) Probing the surface of eukaryotic cells using combinatorial toxin libraries. Current Biology 11, 697-701). Briefly, the strategy to screen all our A chain libraries was based on the following principles. Established cancer cell lines such as SK-BR-3 (human breast), CAMA-1 (human breast), 518A2 (human melanoma), PC3 (human prostate) and B16 (murine melanoma) were grown in 96-well plates and used as targets in the primary screen stages. These cell lines were initially selected for our holotoxin library searches (Bray, supra) based on their adherence (plastic), their cell viability staining properties (SRB) in a high-throughput screening setting as well as their lack ofreceptor and sensitivity to native SLT-1 (to insure a reduced level of false positives). Single bacterial colonies from each library were picked and grown in 96 deep well plates. The cells were harvested, lysed, and their lysates clarified. Since all expressed SLT-1 A chain variants have a 6 histidine tag at their N-terminus, each of them was purified from their lysate using nickel-affinity beads (96-well format) and layered on target cells. The plates containing the target cells treated with A chain variants were then incubated at 37°C for 48 hours, followed by fixation and staining with Sulforhodamine B (SRB). The SRB assay is a calorimetric end-point assay, which quantifies viable cells by staining their cellullar protein content (Skehan, et al. (1990) New colorimetric cytotoxicity assay for anticancer-drug screening. J Natl Cancer Inst 82, 1107-1112.). The SRB assay has been adopted by NCI/NIH for their high-throughput screening of drug candidates on cancer cell lines. Viability assays were repeated for any bacterial extracts leading to cell death. A first round of screening was performed on more than 5000 bacterial clones (equivalent to 5000 distinct A chain toxins) and 7 toxin variants were identified as repeatable killers of the human melanoma cell line 518A2 (Fig. 5). The abscissa represents the log concentration of toxin used to treat the cells and the ordinate depicts the observed percentage of cells that are viable after 48 hours. The closed triangles depicts the effect of the wild type toxin on 518A2 cells while the two most efficacious A chain variants were termed SAM#3 (open squares) and SAM#5 (X symbols).

Seven promising A chain variants were then re-screened against a panel of cell lines (Vero [Monkey, normal kidney]; PC-3 [Human, prostate cancer]; HepG2 [Human, hepatoma]; SiHa [Human, cervical cancer]; PanC [Human, pancreatic cancer]; SKBR-3 [Human, breast cancer]; 518-A2 [Human, melanoma]; U87 [Human, glioma]; B16-F10 [Mouse, melanoma]; HS-216 [Human, normal fibroblast]; CAMA-1 [Human, breast cancer]; OVCar-3 [Human, ovarian cancer]). Of these seven four were observed to observed to have activity against a cancer cell line, two for 518-A2 human melanoma, and one each for SiHa (human cervical cancer cells) and U87-A (human brain cancer cells; glioma).

The genes coding for the two A chain toxins (SAM3 and SAM5) that resulted in toxicity of the human melanoma cell lines were sequenced to determine the amino acid sequences inserted between residues 245 and 246 of the witd-type A chain. The sequences, including the His-tag, are listed in Seq. ID Nos 4 and 5, respectively.

**Table 1: DNA sequences of randomly picked clones from the SLT-1 A chain-tripeptide library. Mutated bases in bold characters. Mutant #41 was identified in our ELISA screen as a strong binder of mAb Onc M27 (Fig. 3).**

| SLT-1 A chainNucleotide sequence | | Diversity |
|---|---|---|
| variant | | (nucleotide changes in mutated region) |
| MUC1 epitope | CCA GAC ACG CGA CCA GCT CCA | 0/9 |
| Mutant #1 | CCA GAC GGG ATC GGG GCT CCA | 8/9 |
| Mutant #2 | CCA GAC CTG GAG ATG GCT CCA | 8/9 |
| Mutant #3 | CCA GAC CCC CGT GGG GCT CCA | 6/9 |
| Mutant #4 | CCA GAC GAC GAC TTG GCT CCA | 9/9 |
| Mutant #5 | CCA GAC GTC CGG TGG GCT CCA | 7/9 |
| Mutant #6 | CCA GAC CAG CGC TGG GCT CCA | 6/9 |
| Mutant #7 | CCA GAC CTC AGG ATG GCT CCA | 8/9 |
| Mutant #8 | CCA GAC TCC CAG GAG GCT CCA | 7/9 |
| Mutant #9 | CCA GAC TCC GAC CCC GCT CCA | 6/9 |
| Mutant #41 | CCA GAC ACG CGC CCC GCT CCA | 2/9 |

**Table 2: Amino acid sequence alignment of randomly selected clones from the SLT-1 A chain-tripeptide library and ELISA signal of purified SLT-1 A chain variants detected with a mAb (Onc M27) raised against the MUC1 epitope Thr-Arg-Pro. Mutated tripeptide region in bold characters. Mutant #41 was identified in our ELISA screen as a strong binder of mAb Onc M27.**

| SLT-1 A chainDeduced amino acid sequence | | ELISA readings |
|---|---|---|
| variant | | (405 nm) |
| MUC1 epitope | CHHHPD TRP APASRVARMASDEFPSMC | 1.3 |
| Mutant #1 | CHHHPD GIG APASRVARMASDEFPSMC | 0.08 |
| Mutant #2 | CHHHPD LQM APASRVARMASDEFPSMC | 0.03 |
| Mutant #3 | CHHHPD PRG APASRVARMASDEFPSMC | 0.03 |
| Mutant #4 | CHHHPD DDL APASRVARMASDEFPSMC | 0.06 |
| Mutant #5 | CHHHPD VRW APASRVARMASDEFPSMC | 0.07 |
| Mutant #6 | CHHHPD QRL APASRVARMASDEFPSMC | 0.06 |
| Mutant #7 | CHHHPD LRM APASRVARMASDEFPSMC | 0.11 |
| Mutant #8 | CHHHPD SQE APASRVARMASDEFPSMC | 0.13 |
| Mutant #9 | CHHHPD SDP APASRVARMASDEFPSMC | 0.07 |
| Mutant #41 | CHHHPD TRP APASRVARMASDEFPSMC | 1.25 |

## Claims

1. A combinatorial protein library comprising a plurality of protein species, each protein species comprising an A chain of an ABx toxic protein comprising a protease-sensitive loop into which an insert has been introduced, wherein the insert is a polypeptide of varying amino acid sequence having a length of 2-7 amino acid residues, and wherein when the plurality of protein species is contacted with at least one candidate target/receptor, a protein species comprising an insert binds to a specific target/receptor, thereby identifying the insert as a ligand of the specific target/receptor.

2. A combinatorial protein library comprising a plurality of protein species, each protein species comprising an A chain of an ABx toxic protein comprising a protease-sensitive loop into which an insert has been introduced, wherein the insert is a polypeptide of varying amino acid sequence having a length of 2-7 amino acid residues, and wherein when the plurality of protein species is contacted with at least one candidate target/receptor located on the surface of a cell, a protein species comprising an insert binds to a specific target/receptor and is toxic to the cell, thereby identifying an insert that comprises a target/receptor ligand.

3. The combinatorial protein library of claim 1, wherein the specific target/receptor is located on the surface of a cell.

4. The combinatorial protein library of any one of claims 1-3, wherein the library comprises at least 100 protein species.

5. The combinatorial protein library of any one of claims 1-4, wherein the protein species are formed by introducing the insert into a Shiga-like toxin I A chain, comprising the amino acid sequence of SEQ ID NO. 1.

6. The combinatorial protein library of claim 5, wherein the protein species is formed by introducing the insert between amino acids 242 and 261, as defined with reference to SEQ ID NO.1.

7. The combinatorial protein library of claim 6, wherein the protein species is formed by introducing the insert between amino acids 245 and 246, as defined with reference to SEQ ID NO. 1.

8. The combinatorial protein library of claim 5, wherein the protein species is formed by introducing the insert before or after amino acids 1-239 of the Shiga-like toxin 1 A chain, as defined with reference to SEQ ID NO. 1.

9. The combinatorial protein library of any one of claims 1-8, wherein the insert has a length of 7 amino acids.

10. A combinatorial expression library comprising a plurality of species of expression systems, each species expressing a protein species in accordance with any one of claims 1-9.

11. A mutant protein comprising an A chain of an ABx toxic protein into which an insert has been introduced, wherein,
(a) the insert is a polypeptide of varying amino acid sequence having a length of 2-7 amino acid residues; and
(b) the insert is introduced into the protease-sensitive loop of the A chain sequence.

12. The mutant protein of claim 11, wherein the A chain of the ABxtoxic protein is a Shiga-like toxin I A chain.

13. The mutant protein of claim 12, wherein the insert is introduced between amino acids 242 and 261, as defined with reference to SEQ ID NO. 1.

14. The mutant protein of claim 13, wherein the insert is introduced between amino acids 245 and 246, as defined with reference to SEQ ID NO. 1.

15. The mutant protein of claim 14, wherein the insert comprises the sequence IYSNKLM (SEQ ID NO. 6).

16. The mutant protein of claim 14, wherein the insert comprises the sequence AAFADLI (SEQ ID NO. 7).

17. The mutant protein of claim 12, wherein the insert is introduced before or after amino acids 1-239 of the Shiga-like toxin I A chain as defined with reference to SEQ ID NO. 1.

18. The mutant protein of any of claims 11-17, wherein the insert has a length of 7 amino acids.

19. A method for identifying a ligand that binds to a specific target/receptor, comprising the steps of:
(a) exposing cells known to possess the target/receptor to members of a combinatorial protein library in accordance with any of claims 1-9; and
(b) selecting members of the protein library which are observed to be toxic to the cells.

20. The method of claim 19, further comprising the step of evaluating the selected members of the protein library to determine the sequence of the inserted region, whereby a peptide of the sequence of the inserted region is identified as a possible ligand for a target/receptor on the cell.

21. The method of any one of claims 19-20, further comprising the step of further testing the selected members of the protein library to confirm that they are ligands for the specific target/receptor.

22. A method for isolating a toxin specific for a target/receptor comprising the steps of:
(a) exposing the target/receptor to a combinatorial protein library according to any one of claims 1-9; and
(b) isolating at least one protein species from the combinatorial protein library captured by binding to the target/receptor.

23. The method of claim 22, further comprising the step of screening the isolated protein against cells expressing the target/receptor to confirm their toxicity for cells expressing the target/receptor.

24. The method of claim 22, wherein the target/receptor is a purified target/receptor and is immobilized on a solid support.

25. The method of claim 23, wherein the target/receptor is on the surface of cells.

26. The method of claim 25, wherein the cells are immobilized on a solid support.

27. The method of any one of claims 25-26, wherein the toxin serves as a reporter, and the death of the cells is indicative of receptor binding.

28. A method for identifying a toxin for a cell expressing a specific target/receptor, comprising the steps of:
(a) exposing cells known to possess the target/receptor to members of a combinatorial protein library in accordance with any one of claims 1-9; and
(b) selecting members of the protein library that are observed to be toxic to the cells, wherein members of the protein library that are observed to be toxic to the cells are toxins for the cells expressing the specific target/receptor.

29. The method of claim 28, further comprising the step of identifying a ligand that binds to a specific target/receptor, comprising the steps of:
(a) exposing cells known to possess the target/receptor to members of a combinatorial protein library in accordance with any one of claims 1-9; and
(b) selecting members of the protein library that are observed to be toxic to the cells, wherein members of the protein library that are observed to be toxic to the cells are ligands that bind to the specific target/receptor.

30. The method of any one of claims 28-29, further comprising a step or steps of mutating an insert to further increase the toxicity of the protein species compared to the protein species comprising the original insert.

31. A method of increasing the toxicity of a protein species of claim 11, comprising the steps of:
(a) mutating a known insert possessing toxicity to cells expressing a specific target/receptor; and
(b) comparing toxicity of the protein species comprising the mutated insert to the protein species comprising the original insert.

32. The method of any one of claims 28-31, further comprising a step of evaluating the selected members of the protein library to determine the sequence of the inserted region.

33. The method of any one of claims 28-32, further comprising a step of further testing the selected members of the protein library to confirm that they are toxic to a cell expressing a specific target/receptor.

34. The method of any one of claims 19-21, 23, or 25-33, wherein the cells are cancer cells.

35. The method of claim 34, wherein the cancer cells are selected from the group consisting of melanoma cells, cervical cancer cells, or brain cancer cells.

36. The method of claim 35, wherein the cancer cells are melanoma cells.

37. The method of claim 35, wherein the cancer cells are cervical cancer cells.

38. The method of claim 35, wherein the cancer cells are brain cancer cells.

## Patentansprüche

1. Kombinatorische Proteinbibliothek, umfassend eine Vielzahl von Proteinarten, wobei jede Proteinart eine A-Kette eines ABx-toxischen Proteins umfasst, die eine Protease-sensitive Schleife umfasst, in die ein Insert eingeführt wurde, wobei das Insert ein Polypeptid mit variierender Aminosäuresequenz ist, die eine Länge von 2 bis 7 Aminosäureresten aufweist, und wobei, wenn die Vielzahl von Proteinarten mit mindestens einem Ziel-/Rezeptor-Kandidaten in Kontakt gebracht wird, eine Proteinart, die ein Insert umfasst, an ein spezifisches Ziel/einen spezifischen Rezeptor bindet, wodurch das Insert als ein Ligand des spezifischen Ziels/Rezeptors identifiziert wird.

2. Kombinatorische Proteinbibliothek, umfassend eine Vielzahl von Proteinarten, wobei jede Proteinart eine A-Kette eines ABx-toxischen Proteins umfasst, die eine Protease-sensitive Schleife umfasst, in die ein Insert eingeführt wurde, wobei das Insert ein Polypeptid mit variierender Aminosäuresequenz ist, die eine Länge von 2 bis 7 Aminosäurereste aufweist, und wobei, wenn die Vielzahl der Proteinarten mit mindestens einem Ziel-/Rezeptor-Kandidaten, der auf der Oberfläche der Zelle lokalisiert ist, in Kontakt gebracht wird, eine Proteinart, die ein Insert umfasst, an ein spezifisches Ziel/einen spezifischen Rezeptor bindet und für die Zelle toxisch ist, wodurch ein Insert, das einen Ziel-/Rezeptorliganden umfasst, identifiziert wird.

3. Kombinatorische Proteinbibliothek nach Anspruch 1, wobei das spezifische Ziel/der spezifische Rezeptor auf der Oberfläche einer Zelle lokalisiert ist.

4. Kombinatorische Proteinbibliothek nach einem der Ansprüche 1 bis 3, wobei die Bibliothek mindestens 100 Proteinarten umfasst.

5. Kombinatorische Proteinbibliothek nach einem der Ansprüche 1 bis 4, wobei die Proteinarten gebildet werden, indem das Insert in eine Shiga-ähnliche Toxin I-A-Kette eingeführt wird, die die Aminosäuresequenz von SEQ ID NO:1 umfasst.

6. Kombinatorische Proteinbibliothek nach Anspruch 5, wobei die Proteinart gebildet wird, indem das Insert zwischen den Aminosäuren 242 und 261 wie für SEQ ID NO:1 definiert eingeführt wird.

7. Kombinatorische Proteinbibliothek nach Anspruch 6, wobei die Proteinart gebildet wird, indem das Insert zwischen den Aminosäuren 245 und 246 wie für SEQ ID NO:1 definiert eingefügt wird.

8. Kombinatorische Proteinbibliothek nach Anspruch 5, wobei die Proteinart gebildet wird, indem das Insert vor oder nach den Aminosäuren 1-239 der Shiga-ähnlichen Toxin I-A-Kette wie für SEQ ID NO:1 definiert eingeführt wird.

9. Kombinatorische Proteinbibliothek nach einem der Ansprüche 1 bis 8, wobei das Insert eine Länge von 7 Aminosäuren aufweist.

10. Kombinatorische Expressionsbibliothek, umfassend eine Vielzahl von Expressionssystemarten, wobei jede Art eine Proteinart nach einem der Ansprüche 1 bis 9 exprimiert.

11. Proteinmutante, umfassend eine A-Kette eines ABx-toxischen Proteins, in das ein Insert eingeführt wurde, wobei
(a) das Insert ein Polypeptid mit variierender Aminosäuresequenz ist, das eine Länge von 2 bis 7 Aminosäureresten aufweist; und
(b) das Insert in die Protease-sensitive Schleife der A-Kettensequenz eingeführt ist.

12. Proteinmutante nach Anspruch 11, wobei die A-Kette des ABx-toxischen Proteins eine Shiga-ähnliche Toxin I-A-Kette ist.

13. Proteinmutante nach Anspruch 12, wobei das Insert zwischen den Aminosäuren 242 und 261 wie für SEQ ID NO:1 definiert eingeführt wird.

14. Proteinmutante nach Anspruch 13, wobei das Insert zwischen den Aminosäuren 245 und 246 wie für SEQ ID NO:1 definiert eingeführt wird.

15. Proteinmutante nach Anspruch 14, wobei das Insert die Sequenz IYSNKLM (SEQ ID NO:6) umfasst.

16. Proteinmutante nach Anspruch 14, wobei das Insert die Sequenz AAFADLI (SEQ ID NO:7) umfasst.

17. Proteinmutante nach Anspruch 12, wobei das Insert vor oder nach den Aminosäuren 1-239 der Shiga-ähnlichen Toxin I-A-Kette wie für SEQ ID NO:1 1 definiert eingeführt wird.

18. Proteinmutante nach einem der Ansprüche 11 bis 17, wobei das Insert eine Länge von 7 Aminosäuren aufweist.

19. Verfahren zum Identifizieren eines Liganden, der an ein spezifisches Ziel/einen spezifischen Rezeptor bindet, umfassend die Schritte:
(a) Aussetzen von Zellen, von denen bekannt ist, dass sie das Ziel/den Rezeptor aufweisen, gegenüber Mitgliedern einer kombinatorischen Proteinbibliothek nach einem der Ansprüche 1 bis 9; und
(b) Auswählen von Mitgliedern der Proteinbibliothek, bei denen Toxizität für die Zellen festgestellt wird.

20. Verfahren nach Anspruch 19, des Weitem umfassend den Schritt der Evaluierung der ausgewählten Mitglieder der Proteinbibliothek, um die Sequenz der insertierten Region zu bestimmen, wobei ein Peptid der Sequenz der insertierten Region als ein möglicher Ligand für ein Ziel/einen Rezeptor auf der Zelle identifiziert wird.

21. Verfahren nach einem der Ansprüche 19 bis 20, des Weiteren umfassend den Schritt, die ausgewählten Mitglieder der Proteinbibliothek weiter zu testen, um zu bestätigen, dass sie Liganden für das spezifische Ziel/den spezifischen Rezeptor sind.

22. Verfahren zum Isolieren eines für ein Ziel/einen Rezeptor spezifischen Toxins, umfassend die Schritte:
(a) Aussetzen des Ziels/Rezeptors einer kombinatorischen Proteinbibliothek nach einem der Ansprüche 1 bis 9; und
(b) Isolieren mindestens einer Proteinart aus der kombinatorischen Proteinbibliothek, die mittels Bindung an das Ziel/den Rezeptor eingefangen wird.

23. Verfahren nach Anspruch 22, des Weiteren umfassend den Schritt, das isolierte Protein gegen Zellen, die das Ziel/den Rezeptor exprimieren, zu screenen, um ihre Toxizität für Zellen, die das Ziel/den Rezeptor exprimieren, zu bestätigen.

24. Verfahren nach Anspruch 22, wobei das Ziel/der Rezeptor ein aufgereinigtes Ziel/aufgereinigter Rezeptor ist und auf einem festen Träger immobilisiert ist.

25. Verfahren nach Anspruch 23, wobei das Ziel/der Rezeptor sich auf der Oberfläche von Zellen befindet.

26. Verfahren nach Anspruch 25, wobei die Zellen auf einem festen Träger immobilisiert sind.

27. Verfahren nach einem der Ansprüche 25 bis 26, wobei das Toxin als ein Reporter dient und der Zelltod ein Indikator für die Rezeptorbindung ist.

28. Verfahren zum Identifizieren eines Toxins für eine Zelle, die ein spezifisches Ziel/einen spezifischen Rezeptor exprimiert, umfassend die Schritte:
(a) Aussetzen von Zellen, von denen bekannt ist, dass sie das Ziel/den Rezeptor aufweisen, gegenüber Mitgliedern einer kombinatorischen Proteinbibliothek nach einem der Ansprüche 1 bis 9; und
(b) Auswählen von Mitgliedern der Proteinbibliothek, von denen festgestellt wird, dass sie toxisch für die Zellen sind,
wobei die Mitglieder der Proteinbibliothek, von denen festgestellt wird, dass sie toxisch für die Zellen sind, Toxine für die Zellen sind, die das spezifische Ziel/den spezifischen Rezeptor exprimieren.

29. Verfahren nach Anspruch 28, des Weiteren umfassend den Schritt, einen Liganden zu identifizieren, der an ein spezifisches Ziel/einen spezifischen Rezeptor bindet, umfassend die Schritte:
(a) Aussetzen der Zellen, von denen bekannt ist, dass sie das Ziel/den Rezeptor aufweisen, gegenüber Mitgliedern einer kombinatorischen Proteinbibliothek nach einem der Ansprüche 1 bis 9; und
(b) Auswählen der Mitglieder der Proteinbibliothek, von denen festgestellt wird, dass sie toxisch für die Zellen sind,
wobei die Mitglieder der Proteinbibliothek, von denen festgestellt wird, dass sie toxisch für die Zellen sind, Liganden sind, die an das spezifische Ziel/den spezifischen Rezeptor binden.

30. Verfahren nach einem der Ansprüche 28 bis 29, des Weiteren umfassend einen Schritt oder Schritte des Mutierens eines Inserts, um die Toxizität der Proteinart im Vergleich mit der Proteinart, die das ursprüngliche Insert umfasst, zu erhöhen.

31. Verfahren zum Erhöhen der Toxizität einer Proteinart nach Anspruch 11, umfassend die Schritte:
(a) Mutieren eines bekannten Inserts, das Toxizität für Zellen aufweist, die ein spezifisches Ziel/einen spezifischen Rezeptor exprimieren; und
(b) Vergleichen der Toxizität der Proteinart, die das mutierte Insert umfasst, mit der Proteinart, die das ursprüngliche Insert umfasst.

32. Verfahren nach einem der Ansprüche 28 bis 31, des Weiteren umfassend einen Schritt der Evaluierung der ausgewählten Mitglieder der Proteinbibliothek, um die Sequenz der insertierten Region zu bestimmen.

33. Verfahren nach einem der Ansprüche 28 bis 32, des Weiteren umfassend einen Schritt, die ausgewählten Mitglieder der Proteinbibliothek weiter zu testen, um zu bestätigen, dass sie für eine Zelle, die ein spezifisches Ziel/einen spezifischen Rezeptor exprimiert, toxisch ist.

34. Verfahren nach einem der Ansprüche 19 bis 21, 23 oder 25 bis 33, wobei die Zellen Krebszellen sind.

35. Verfahren nach Anspruch 34, wobei die Krebszellen ausgewählt sind aus der Gruppe bestehend aus Melanomzellen, Gebärmutterhalskrebszellen oder Hirnkrebszellen.

36. Verfahren nach Anspruch 35, wobei die Krebszellen Melanomzellen sind.

37. Verfahren nach Anspruch 35, wobei die Krebszellen Gebärmutterhals-krebszellen sind.

38. Verfahren nach Anspruch 35, wobei die Krebszellen Hirnkrebszellen sind.

## Revendications

1. Banque combinatoire de protéines comprenant une pluralité d'espèces de protéines, chaque espèce de protéine comprenant une chaîne A d'une protéine toxique ABx comprenant une boucle sensible aux protéases dans laquelle un insert a été introduit, où l'insert est un polypeptide de séquence d'acides aminés variable ayant une longueur de 2 à 7 résidus d'acides aminés, et où lorsque la pluralité des espèces de protéines est mise en contact avec au moins une cible/un récepteur candidat, une espèce de protéine comprenant un insert se lie à une cible/un récepteur spécifique, identifiant de cette manière l'insert comme un ligand de la cible/du récepteur spécifique.

2. Banque combinatoire de protéines comprenant une pluralité d'espèces de protéines, chaque espèce de protéine comprenant une chaîne A d'une protéine toxique ABx comprenant une boucle sensible aux protéases dans laquelle un insert a été introduit, où l'insert est un polypeptide de séquence d'acides aminés variable ayant une longueur de 2 à 7 résidus d'acides aminés, et où lorsque la pluralité des espèces de protéines est mise en contact avec au moins une cible/un récepteur candidat localisé(e) sur la surface d'une cellule, une espèce de protéine comprenant un insert se lie à une cible/un récepteur spécifique et est toxique envers la cellule, identifiant de cette manière un insert qui comprend un ligand de la cible/du récepteur.

3. Banque combinatoire de protéines selon la revendication 1, dans laquelle la cible/le récepteur spécifique est localisé(e) sur la surface d'une cellule.

4. Banque combinatoire de protéines selon l'une quelconque des revendications 1 à 3, où la banque comprend au moins 100 espèces de protéines.

5. Banque combinatoire de protéines selon l'une quelconque des revendications 1 à 4, dans laquelle les espèces de protéines sont formées en introduisant l'insert dans une chaîne A de toxine I de type Shiga, comprenant la séquence d'acides aminés de SEQ ID NO : 1.

6. Banque combinatoire de protéines selon la revendication 5, dans laquelle l'espèce de protéine est formée en introduisant l'insert entre les acides aminés 242 et 261, comme il est défini en référence à SEQ ID NO : 1.

7. Banque combinatoire de protéines selon la revendication 6, dans laquelle l'espèce de protéine est formée en introduisant l'insert entre les acides aminés 245 et 246, comme il est défini en référence à SEQ ID NO : 1.

8. Banque combinatoire de protéines selon la revendication 5, dans laquelle l'espèce de protéine est formée en introduisant l'insert avant ou après les acides aminés 1 à 239 de la chaîne A de la toxine I de type Shiga, comme il est défini en référence à SEQ ID NO : 1.

9. Banque combinatoire de protéines selon l'une quelconque des revendications 1 à 8, dans laquelle l'insert a une longueur de 7 acides aminés.

10. Banque combinatoire d'expression comprenant une pluralité d'espèces de systèmes d'expression, chaque espèce exprimant une espèce de protéine conformément à l'une quelconque des revendications 1 à 9.

11. Protéine mutante comprenant une chaîne A d'une protéine toxique ABx dans laquelle un insert a été introduit, où,
(a) l'insert est un polypeptide de séquence d'acides aminés variable ayant une longueur de 2 à 7 résidus d'acides aminés ; et
(b) l'insert est introduit dans la boucle sensible aux protéases de la séquence de la chaîne A.

12. Protéine mutante selon la revendication 11, dans laquelle la chaîne A de la protéine toxique ABx est une chaîne A de la toxine I de type Shiga.

13. Protéine mutante selon la revendication 12, dans laquelle l'insert est introduit entre les acides aminés 242 et 261, comme il est défini en référence à SEQ ID NO : 1.

14. Protéine mutante selon la revendication 13, dans laquelle l'insert est introduit entre les acides aminés 245 et 246, comme il est défini en référence à SEQ ID NO : 1.

15. Protéine mutante selon la revendication 14, dans laquelle l'insert comprend la séquence IYSNKLM (SEQ ID NO : 6).

16. Protéine mutante selon la revendication 14, dans laquelle l'insert comprend la séquence AAFADLI (SEQ ID NO : 7).

17. Protéine mutante selon la revendication 12, dans laquelle l'insert est introduit avant ou après les acides aminés 1 à 239 de la chaîne A de la toxine I de type Shiga, comme il est défini en référence à SEQ ID NO : 1.

18. Protéine mutante selon l'une quelconque des revendications 11 à 17, dans laquelle l'insert a une longueur de 7 acides aminés.

19. Procédé d'identification d'un ligand qui se lie à une cible/un récepteur spécifique, comprenant les étapes suivantes :
(a) l'exposition des cellules connues pour posséder la cible/le récepteur à des membres d'une banque combinatoire de protéines conformément à l'une quelconque des revendications 1 à 9 ; et
(b) le choix de membres de la banque de protéines qui sont observés comme étant toxiques envers les cellules.

20. Procédé selon la revendication 19, comprenant en outre l'étape d'évaluation des membres choisis de la banque de protéines pour déterminer la séquence de la région insérée, moyennant quoi un peptide de la séquence de la région insérée est identifié comme étant un ligand possible pour une cible/un récepteur sur la cellule.

21. Procédé selon l'une quelconque des revendications 19 à 20, comprenant en outre l'étape consistant à tester en outre les membres choisis de la banque de protéines pour confirmer que ce sont des ligands pour la cible/le récepteur spécifique.

22. Procédé pour isoler une toxine spécifique d'une cible/d'un récepteur comprenant les étapes suivantes :
(a) l'exposition de la cible/du récepteur à une banque combinatoire de protéines selon l'une quelconque des revendications 1 à 9 ; et
(b) l'isolement d'au moins une espèce de protéine dans la banque combinatoire de protéines capturée par la liaison à la cible/au récepteur.

23. Procédé selon la revendication 22, comprenant en outre l'étape de criblage de la protéine isolée contre les cellules exprimant la cible/le récepteur pour confirmer leur toxicité pour des cellules exprimant la cible/le récepteur.

24. Procédé selon la revendication 22, dans lequel la cible/le récepteur est une cible/un récepteur purifié(e) et est immobilisé(e) sur un support solide.

25. Procédé selon la revendication 23, dans lequel la cible/le récepteur est sur la surface des cellules.

26. Procédé selon la revendication 25, dans lequel les cellules sont immobilisées sur un support solide.

27. Procédé selon l'une quelconque des revendications 25 à 26, dans lequel la toxine sert de rapporteur et la mort des cellules indique la liaison au récepteur.

28. Procédé pour identifier une toxine pour une cellule exprimant une cible/un récepteur spécifique, comprenant les étapes suivantes :
(a) l'exposition des cellules connues pour posséder la cible/le récepteur à des membres d'une banque combinatoire de protéines conformément à l'une quelconque des revendications 1 à 9 ; et
(b) le choix de membres de la banque de protéines qui sont observés comme étant toxiques envers les cellules
dans lequel les membres de la banque de protéines qui sont observés comme étant toxiques envers les cellules sont des toxines pour les cellules exprimant la cible/le récepteur spécifique.

29. Procédé selon la revendication 28, comprenant en outre l'étape d'identification d'un ligand qui se lie à une cible/un récepteur spécifique, comprenant les étapes suivantes :
(a) l'exposition des cellules connues pour posséder la cible/le récepteur à des membres d'une banque combinatoire de protéines conformément à l'une quelconque des revendications 1 à 9 ; et
(b) le choix de membres de la banque de protéines qui sont observés comme étant toxiques envers les cellules
dans lequel les membres de la banque de protéines qui sont observés comme étant toxiques envers les cellules sont des ligands qui se lient à la cible/au récepteur spécifique.

30. Procédé selon l'une quelconque des revendications 28 à 29, comprenant en outre une étape ou des étapes de mutation d'un insert pour encore augmenter la toxicité de l'espèce de protéine comparativement à l'espèce de protéine comprenant l'insert d'origine.

31. Procédé d'augmentation de la toxicité d'une espèce de protéine selon la revendication 11, comprenant les étapes suivantes :
(a) la mutation d'un insert connu comme possédant une toxicité envers les cellules exprimant une cible/un récepteur spécifique ; et
(b) la comparaison de la toxicité de l'espèce de protéine comprenant l'insert muté à l'espèce de protéine comprenant l'insert d'origine.

32. Procédé selon l'une quelconque des revendications 28 à 31, comprenant en outre une étape d'évaluation des membres choisis de la banque de protéines pour déterminer la séquence de la région insérée.

33. Procédé selon l'une quelconque des revendications 28 à 32, comprenant en outre une étape consistant à tester en outre les membre choisis de la banque de protéines pour confirmer qu'ils sont toxiques envers une cellule exprimant une cible/un récepteur spécifique.

34. Procédé selon l'une quelconque des revendications 19 à 21, 23 ou 25 à 33, dans lequel les cellules sont des cellules cancéreuses.

35. Procédé selon la revendication 34, dans lequel les cellules cancéreuses sont choisies dans le groupe constitué de cellules de mélanome, de cellules de cancer du col de l'utérus ou de cellules de cancer du cerveau.

36. Procédé selon la revendication 35, dans lequel les cellules cancéreuses sont des cellules de mélanome.

37. Procédé selon la revendication 35, dans lequel les cellules cancéreuses sont des cellules de cancer du col de l'utérus.

38. Procédé selon la revendication 35, dans lequel les cellules cancéreuses sont des cellules de cancer du cerveau.


### SEQUENCE LISTING

<110> Molecular Templates, Inc.
<120> LIBRARY OF TOXIN MUTANTS, AND METHODS OF USING SAME
<130> M2798 EP/1 S3
<150> EP 04 78 5852.7
   <151> 2004-03-26
<160> 7
<170> PatentIn version 3.2
<210> 1
   <211> 299
   <212> PRT
   <213> Escherichia coli
<220>
   <221> misc_feature
   <223> Wild type SLT-1 A chain
<400> 1
   Ile Glu Gly Arg Ala Ser Lys Glu Phe Thr Leu Asp Phe Ser Thr Ala
   1               5                   10                  15
   Lys Thr Tyr Val Asp Ser Leu Asn Val Ile Arg Ser Ala Ile Gly Thr
               20                  25                  30
   Pro Leu Gln Thr Ile Ser Ser Gly Gly Thr Ser Leu Leu Met Ile Asp
           35                  40                  45
   Ser Gly Ser Gly Asp Asn Leu Phe Ala Val Asp Val Arg Gly Ile Asp
       50                  55                  60
   Pro Glu Glu Gly Arg Phe Asn Asn Leu Arg Leu Ile Val Glu Arg Asn
   65                  70                  75                  80
   Asn Leu Tyr Val Thr Gly Phe Val Asn Arg Thr Asn Asn Val Phe Tyr
                   85                  90                  95
   Arg Phe Ala Asp Phe Ser His Val Thr Phe Pro Gly Thr Thr Ala Val
               100                 105                 110
   Thr Leu Ser Gly Asp Ser Ser Tyr Thr Thr Leu Gln Arg Val Ala Gly
           115                 120                 125
   Ile Ser Arg Thr Gly Met Gln Ile Asn Arg His Ser Leu Thr Thr Ser
       130                 135                 140
   Tyr Leu Asp Leu Met Ser His Ser Gly Thr Ser Leu Thr Gln Ser Val
   145                 150                 155                 160
   Ala Arg Ala Met Leu Arg Phe Val Thr Val Thr Ala Glu Ala Leu Arg
                   165                 170                 175
   Phe Arg Gln Ile Gln Arg Gly Phe Arg Thr Thr Leu Asp Asp Leu Ser
               180                 185                 190
   Gly Arg Ser Tyr Val Met Thr Ala Glu Asp Val Asp Leu Thr Leu Asn
           195                 200                 205
   Trp Gly Arg Leu Ser Ser Val Leu Pro Asp Tyr His Gly Gln Asp Ser
       210                 215                 220
   Val Arg Val Gly Arg Ile Ser Phe Gly Ser Ile Asn Ala Ile Leu Gly
   225                 230                 235                 240
   Ser Val Ala Leu Ile Leu Asn Cys His His His Ala Ser Arg Val Ala
                   245                 250                 255
   Arg Met Ala Ser Asp Glu Phe Pro Ser Met Cys Pro Ala Asp Gly Arg
               260                 265                 270
   Val Arg Gly Ile Thr His Asn Lys Ile Leu Trp Asp Ser Ser Thr Leu
           275                 280                 285
   Gly Ala Ile Leu Met Arg Arg Thr Ile Ser Ser
       290                 295
<210> 2
   <211> 32
   <212> DNA
   <213> Artificial
<220>
   <221> Source
   <223> /note = "Description of Artificial Sequence:Primer"
<400> 2
   gttactgtga cagctgaagc tttacgtttt cg 32
<210> 3
   <211> 31
   <212> DNA
   <213> Artificial
<220>
   <221> Source
   <223> /note = "Description of Artificial Sequence:Primer"
<400> 3
   gagaagaaga gactgcagat tccatctgtt g 31
<210> 4
   <211> 302
   <212> PRT
   <213> Artificial
<220>
   <221> Source
   <223> /note = "Description of Artificial Sequence: SLT-1 A Chain lib#3 protein sequence (SAM3)"
<400> 4
   Lys Gly Met Arg Ser His His His His His His His His Ile Glu Gly
   1               5                   10                  15
   Arg Ala Ser Lys Glu Phe Thr Leu Asp Phe Ser Thr Ala Lys Thr Tyr
               20                  25                  30
   Val Asp Ser Leu Asn Val Ile Arg Ser Ala Ile Gly Thr Pro Leu Gln
           35                  40                  45
   Thr Ile Ser Ser Gly Gly Thr Ser Leu Leu Met Ile Asp Ser Gly Ser
       50                  55                  60
   Gly Asp Asn Leu Phe Ala Val Asp Val Arg Gly Ile Asp Pro Glu Glu
   65                  70                  75                  80
   Gly Arg Phe Asn Asn Leu Arg Leu Ile Val Glu Arg Asn Asn Leu Tyr
                   85                  90                  95
   Val Thr Gly Phe Val Asn Arg Thr Asn Asn Val Phe Tyr Arg Phe Ala
               100                 105                 110
   Asp Phe Ser His Val Thr Phe Pro Gly Thr Thr Ala Val Thr Leu Ser
           115                 120                 125
   Gly Asp Ser Ser Tyr Thr Thr Leu Gln Arg Val Ala Gly Ile Ser Arg
       130                 135                 140
   Thr Gly Met Gln Ile Asn Arg His Ser Leu Thr Thr Ser Tyr Leu Asp
   145                 150                 155                 160
   Leu Met Ser His Ser Gly Thr Ser Leu Thr Gln Ser Val Ala Arg Ala
                   165                 170                 175
   Met Leu Arg Phe Val Thr Val Thr Ala Glu Ala Leu Arg Phe Arg Gln
               180                 185                 190
   Ile Gln Arg Gly Phe Arg Thr Thr Leu Asp Asp Leu Ser Gly Arg Ser
           195                 200                 205
   Tyr Val Met Thr Ala Glu Asp Val Asp Leu Thr Leu Asn Trp Gly Arg
       210                 215                 220
   Leu Ser Ser Val Leu Pro Asp Tyr His Gly Gln Asp Ser Val Arg Val
   225                 230                 235                 240
   Gly Arg Ile Ser Phe Gly Ser Ile Asn Ala Ile Leu Gly Ser Val Ala
                   245                 250                 255
   Leu Ile Leu Asn Cys His His His Ile Tyr Ser Asn Lys Leu Met Ala
               260                 265                 270
   Ser Arg Val Ala Arg Met Ala Ser Asp Glu Phe Pro Ser Met Cys Pro
           275                 280                 285
   Ala Asp Gly Arg Val Arg Gly Ile Thr His Asn Lys Ile Leu
       290                 295                 300
<210> 5
   <211> 319
   <212> PRT
   <213> Artificial
<220>
   <221> Source
   <223> /note = "Description of Artificial Sequence: SLT-1 A Chain lib#5 protein sequence (SAM5)"
<400> 5
   Lys Gly Met Arg Ser His His His His His His His His Ile Glu Gly
   1               5                   10                  15
   Arg Ala Ser Lys Glu Phe Thr Leu Asp Phe Ser Thr Ala Lys Thr Tyr
               20                  25                  30
   Val Asp Ser Leu Asn Val Ile Arg Ser Ala Ile Gly Thr Pro Leu Gln
           35                  40                  45
   Thr Ile Ser Ser Gly Gly Thr Ser Leu Leu Met Ile Asp Ser Gly Ser
       50                  55                  60
   Gly Asp Asn Leu Phe Ala Val Asp Val Arg Gly Ile Asp Pro Glu Glu
   65                  70                  75                  80
   Gly Arg Phe Asn Asn Leu Arg Leu Ile Val Glu Arg Asn Asn Leu Tyr
                   85                  90                  95
   Val Thr Gly Phe Val Asn Arg Thr Asn Asn Val Phe Tyr Arg Phe Ala
               100                 105                 110
   Asp Phe Ser His Val Thr Phe Pro Gly Thr Thr Ala Val Thr Leu Ser
           115                 120                 125
   Gly Asp Ser Ser Tyr Thr Thr Leu Gln Arg Val Ala Gly Ile Ser Arg
       130                 135                 140
   Thr Gly Met Gln Ile Asn Arg His Ser Leu Thr Thr Ser Tyr Leu Asp
   145                 150                 155                 160
   Leu Met Ser His Ser Gly Thr Ser Leu Thr Gln Ser Val Ala Arg Ala
                   165                 170                 175
   Met Leu Arg Phe Val Thr Val Thr Ala Glu Ala Leu Arg Phe Arg Gln
               180                 185                 190
   Ile Gln Arg Gly Phe Arg Thr Thr Leu Asp Asp Leu Ser Gly Arg Ser
           195                 200                 205
   Tyr Val Met Thr Ala Glu Asp Val Asp Leu Thr Leu Asn Trp Gly Arg
       210                 215                 220
   Leu Ser Ser Val Leu Pro Asp Tyr His Gly Gln Asp Ser Val Arg Val
   225                 230                 235                 240
   Gly Arg Ile Ser Phe Gly Ser Ile Asn Ala Ile Leu Gly Ser Val Ala
                   245                 250                 255
   Leu Ile Leu Asn Cys His His His Ala Ala Phe Ala Asp Leu Ile Ala
               260                 265                 270
   Ser Arg Val Ala Arg Met Ala Ser Asp Glu Phe Pro Ser Met Cys Pro
           275                 280                 285
   Ala Asp Gly Arg Val Arg Gly Ile Thr His Asn Lys Ile Leu Trp Asp
       290                 295                 300
   Ser Ser Thr Leu Gly Ala Ile Leu Met Arg Arg Thr Ile Ser Ser
   305                 310                 315
<210> 6
   <211> 7
   <212> PRT
   <213> Artificial
<220>
   <221> Source
   <223> /note = "Description of Artificial Sequence: First melanoma active insert"
<400> 6
   Ile Tyr Ser Asn Lys Leu Met
   1               5
<210> 7
   <211> 7
   <212> PRT
   <213> Artificial
<220>
   <221> Source
   <223> /note = "Description of Artificial Sequence: Second melanoma active insert"
<400> 7
   Ala Ala Phe Ala Asp Leu Ile
   1               5